# EUROPEAN PATENT APPLICATION

(11) **EP 4 817 613 A1**
(43) Date of publication of application: **07.10.2026**
(21) Application number: 24898143.3
(22) Date of filing: 28.11.2024
(51) Int. Cl.: A61K 31/437, A61P 31/10, A61P 1/04

(54) **COMPOSITION FOR ERADICATING HELICOBACTER PYLORI COMPRISING ZASTAPRAZAN OR PHARMACEUTICALLY ACCEPTABLE SALT THEREOF**

(30) Priority: 29.11.2023 KR 20230169710
(71) Applicant: Jeil Pharmaceutical Co., Ltd., Seoul 06543 (KR); Onconic Therapeutics Inc., Gangnam-gu Seoul 06236 (KR)
(72) Inventor: KIM, John, Seoul 06732 (KR); CHA, Hyun Ju, Seoul 06714 (KR); HAN, Sang Woo, Seoul 06274 (KR)
(74) Representative: Louis Pöhlau Lohrentz
(86) International application number: PCT/KR2024/019081
(87) International publication number: WO 2025/116549

(57) **Abstract**

Disclosed is a composition for eradicating Helicobacter pylori, comprising zastaprazan or a pharmaceutically acceptable salt thereof.

## Description

### [Technical Field]

The present invention relates to a composition for *Helicobacter pylori* (*H. pylori*) eradication, including zastaprazan or a pharmaceutically acceptable salt thereof, more specifically, to a composition for *H. pylori* eradication, including zastaprazan, a pharmaceutically acceptable salt thereof, a hydrate or solvate thereof, or a mixture thereof, wherein the composition exhibits a therapeutic effect on gastroesophageal reflux disease and/or peptic ulcer regardless of *H. pylori* infection due to its own eradicating effect, and rather tends to exhibit a high effect on patients infected with *H. pylori*.

### [Background Art]

*Helicobacter pylori* (*H. pylori*) is a Gram-negative spirillum that lives in the human gastric mucosa, and it is classified as a group 1 carcinogen by the World Health Organization (WHO) and is infected with about half of the adults in Korea. *H. pylori* is a bacterium that parasitizes the gastric mucosa and continues to grow, and it does not disappear on its own unless appropriate treatment is received.

The infection route of the *H. pylori* bacteria is not yet clearly known, but it is known to be transmitted from person to person. It is understood that infection is closely related to the unique lifestyle of Koreans, so they are exposed to the risk of infection with *H. pylori* bacteria.

*H. pylori* has been identified as the cause of atrophic gastritis, intestinal metaplasia, peptic ulcers, gastric mucosa-associated lymphoid tissue (MALT) lymphoma, and gastric cancer, and it is also known to be related to functional dyspepsia, iron deficiency anemia of unknown cause, and chronic idiopathic thrombocytopenia, or the like.

The results of a multicenter study of asymptomatic adults in Korea showed that the serological prevalence rate was 51.0% in 2015, showing a steady decrease compared to the past. This may be attributed to not only active treatment but also the improvement of economic status and sanitary conditions.

The first-line eradication of *H. pylori* is to take a gastric acid secretion inhibitor and two types of antibiotics (amoxicillin and clarithromycin) for 14 days. The treatment success rate is 70% to 80%, but some patients stop taking the medication on their own will, often resulting in treatment failure. In these patients, treatment difficulties may occur due to resistant strains.

Among the pathogenic factors of *H. pylori*, urease neutralizes gastric acid, allowing *H. pylori* to settle and proliferate in a strongly acidic environment. In addition, ammonia, produced as a result of the enzymatic action, affects gastric epithelial cells, and various cytokines produced when *H. pylori* stimulates gastric epithelial cells serve to collect various inflammatory cells.

Therefore, it is necessary to develop a new therapeutic agent that can directly exert an antibacterial effect against *H. pylori* or a urease inhibitory effect.

### [Related Art Documents]

### [Patent Document]

(Patent Document 1) Korean Patent Publication 10-2000-0005291

### [Disclosure]

### [Technical Problem]

The present inventors have confirmed the need for research and development of a new therapeutic agent capable of exerting a direct antibacterial effect against *Helicobacter pylori* (*H. pylori*) or a urease inhibitory effect. Accordingly, an object of the present invention is to provide a composition for bacterial eradication, including zastaprazan, a pharmaceutically acceptable salt thereof, a hydrate or solvate thereof, or a mixture thereof, wherein the composition exhibits a therapeutic effect on gastroesophageal reflux disease and/or peptic ulcer regardless of *H. pylori* infection and is capable of exhibiting a high effect particularly on patients infected with *H. pylori*.

### [Technical Solution]

In order to achieve the above purpose, the present invention discloses the following means.

In one aspect, the present invention provides a composition for *H. pylori* eradication, including zastaprazan, a pharmaceutically acceptable salt thereof, a hydrate or solvate thereof, or a mixture thereof.

### [Advantageous Effects]

The composition for bacterial eradication of the present invention includes zastaprazan, a pharmaceutically acceptable salt thereof, a hydrate or solvate thereof, or a mixture thereof, and the composition has the advantage that it exhibits a therapeutic effect on gastroesophageal reflux disease and/or peptic ulcer regardless of *H. pylori* infection due to its own *H. pylori* eradicating effect and exhibits a high effect particularly on patients infected with *H. pylori*.

In addition, the composition for bacterial eradication of the present invention has the advantages in that, in the evaluation of all symptoms (heartburn, acid reflux, heartburn/acid reflux) appearing due to *H. pylori* infection, all symptoms tend to improve within 24 hours and for 7 days.

The effects of the present invention are not limited to the above-mentioned effects, and various effects may be included within a range obvious to those skilled in the art from the contents described below.

### [Best Mode]

Hereinafter, the present invention will be described in more details.

This is explained specifically as follows. The terms used herein are selected from the most widely used general terms possible while considering the functions in the present invention, but they may vary depending on the intention of those skill in the art, precedents, the emergence of new technologies, or the like. In addition, in certain cases, there are terms arbitrarily selected by the applicant, and in this case, the meanings thereof will be described in detail in the corresponding part of the detailed description of the invention. Therefore, the terms used herein should be defined based on the meanings of the terms and the overall contents of the present invention, rather than simply the names of the terms.

Unless otherwise defined, all terms used herein, including technical and scientific terms, have the same meaning as generally understood by one of ordinary skill in the art to which the present invention pertains. Terms, such as those defined in commonly used dictionaries, should be interpreted as having a meaning that is consistent with their meaning in the context of the relevant art and are not interpreted in an idealized or overly formal sense unless clearly so defined in the present invention.

Numerical ranges are inclusive of the values defined therein. Every maximum numerical limitation given throughout the present specification includes every lower numerical limitation, as if such lower numerical limitations were expressly written. Every minimum numerical limitation given throughout the present specification includes every higher numerical limitation, as if such higher numerical limitations were expressly written. Every numerical limitation given throughout the present specification will include every better numerical range within the broader numerical range, as if the narrower numerical limitations were expressly written.

Hereinafter, each description and embodiment disclosed in the present invention may also be applied to other descriptions and embodiments, respectively. In other words, all combinations of various elements disclosed in the present invention fall within the scope of the present invention. In addition, the scope of the present invention may not be considered as being limited by the specific description described below.

As used herein, expressions such as "including" are to be understood as open-ended terms implying the possibility of including other embodiments, unless specifically stated otherwise in the phrase or sentence in which the expression is included.

The term "zastaprazan" used herein may refer to zastaprazan, a pharmaceutically acceptable salt thereof, a hydrate or solvate thereof, or a mixture thereof. Therefore, "a composition including zastaprazan" as used herein may refer to a composition including zastaprazan, a pharmaceutically acceptable salt thereof, a hydrate or solvate thereof, or a mixture thereof.

Hereinafter, the present invention will be described in detail.

### Composition for H. pylori eradication, including zastaprazan

The present invention discloses a composition for *Helicobacter pylori* (*H. pylori*) eradication, including zastaprazan.

Specifically, the present invention provides a composition for *H. pylori* eradication, including zastaprazan, a pharmaceutically acceptable salt thereof, a hydrate or solvate thereof, or a mixture thereof.

The composition for bacterial eradication of the present invention includes zastaprazan, a pharmaceutically acceptable salt thereof, a hydrate or solvate thereof, or a mixture thereof, and the composition has the advantage that it exhibits a therapeutic effect on gastroesophageal reflux disease and/or peptic ulcer regardless of *H. pylori* infection due to its own *H. pylori* eradicating effect and exhibits a high effect particularly on patients infected with *H. pylori*.

In addition, the composition for bacterial eradication of the present invention exhibits a synergistic effect in the *H. pylori* eradicating effect with the gastric acid secretion inhibition effect (potassium competitive acid blocker; P-CAB) due to the unique reversible proton pump inhibition effect of zastaprazan, thereby exhibiting a higher therapeutic effect in *H. pylori*-infected patients with a gastroesophageal reflux disease or peptic ulcer.

In addition, the composition for bacterial eradication of the present invention has the advantages in that, in the evaluation of all symptoms (heartburn, acid reflux, heartburn/acid reflux) appearing due to *H. pylori* infection, all symptoms tend to improve within 24 hours and for 7 days.

In the present invention, zastaprazan is an example of an imidazo[1,2-a]pyridine derivative, and its chemical name is azetidin-1-yl-[8-[(2,6-dimethylbenzyl)amino]-2,3-dimethylimidazo[1,2-a]pyridin-6-yl]methanone.

In the present invention, zastaprazan is an active ingredient for *H. pylori* eradication.

The term "active ingredient" as used herein refers to a substance or group of substances that are expected to directly or indirectly exhibit the efficacy and effect of the pharmaceutical composition through inherent pharmacological action, and its includes a main ingredient.

The zastaprazan of the present invention may be present in the form of a pharmaceutically acceptable salt, and as a salt, an acid addition salt formed by a pharmaceutically acceptable free acid is useful. The term "pharmaceutically acceptable salt" as used herein refers to any organic or inorganic acid addition salt of the zastaprazan having a concentration that has relatively non-toxic and harmless effect on a patient, and the side effects caused by the salt do not reduce the beneficial efficacy of zastaprazan. Organic acids and inorganic acids may be used as free acids, and inorganic acids such as hydrochloric acid, phosphoric acid, sulfuric acid, nitric acid, or tartaric acid may be used, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid may be used.

Pharmaceutically acceptable salts of the present invention include acidic or basic salts which may be present in zastaprazan, unless otherwise indicated. For example, pharmaceutically acceptable salts may include sodium, calcium and potassium salts of hydroxyl groups, and other pharmaceutically acceptable salts of amino groups include hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, and the like, and may be prepared by methods for preparing salts known in the art.

Specifically, the pharmaceutically acceptable salt of zastaprazan may be a zastaprazan citrate salt (azetidin-1-yl{8-[(2,6-dimethylbenzyl)amino]-2,3-dimethylimidazo[1,2-a]pyridin-6-yl}methanone citrate salt), but is not limited thereto.

The term "hydrate" as used herein refers to a hydrate formed by binding the active ingredient, zastaprazan or a pharmaceutically acceptable salt thereof, to water by non-covalent intermolecular forces, and it contains a stoichiometric or non-stoichiometric amount of water. Specifically, the hydrate may contain water at a ratio of about 0.25 mol to about 10 mol based on 1 mol of the active ingredient, and more specifically, may contain about 0.5 mol, about 1 mol, about 1.5 mol, about 2 mol, about 3 mol, about 5 mol, or the like.

The term "solvate" as used herein refers to a compound formed by binding the active ingredient, zastaprazan or a pharmaceutically acceptable salt thereof, and a solvent by non-covalent intermolecular forces, and it contains a stoichiometric or non-stoichiometric amount of the solvent. Preferred solvents are volatile and non-toxic and may be administered to humans in very small amounts. Examples thereof include methanol, ethanol, 1-propanol, 2-propanol, 1-butanol, 2-butanol, 1-acetate, acetone, acetic acid, anisole, tetrahydrofuran, methyl acetate, ethyl acetate, propyl acetate, isopropyl acetate, isobutyl acetate, n-butyl acetate, dimethyl sulfoxide, pentane, and heptane, but the solvate of the present invention is not limited to these examples, and specifically, the solvate may contain solvent at a ratio of about 0.25 mol to about 10 mol based on 1 mol of the active ingredient, and more specifically, may contain about 0.5 mol, about 1 mol, about 1.5 mol, about 2 mol, about 3 mol, about 5 mol, or the like.

The term "bacterial eradication" as used herein has a similar meaning to sterilization, and means eliminating bacteria. The term means a state in which the metabolism is stopped without directly killing the microorganism until the microorganism dies after a certain period of time, that is, a state in which the proliferation and growth of the bacteria are stopped. The *H. pylori* eradication herein may be considered to include the elimination or eradication of *H. pylori* present in the human stomach, or the stopping of the proliferation and growth of *H. pylori*.

In the present invention, the composition may be administered once a day to three times a day. Specifically, the zastaprazan, a pharmaceutically acceptable salt thereof, a hydrate or solvate thereof, or a mixture thereof may be administered once a day to three times a day for 1 week or more or 4 weeks or more, and may be administered for 24 weeks or less or 12 weeks or less. Specifically, the composition may be administered once a day to three times a day for 1 week to 24 weeks, 2 weeks to 24 weeks, 4 weeks to 24 weeks, 1 week to 12 weeks, 2 weeks to 12 weeks, or 4 weeks to 12 weeks, and preferably, it may be administered for 4 weeks to 8 weeks, but is not limited thereto.

In the present invention, zastaprazan, a pharmaceutically acceptable salt thereof, a hydrate or solvate thereof, or a mixture thereof may be contained in an amount of 1 mg to 100 mg, 2 mg to 60 mg, or 3 mg to 40 mg as zastaprazan (in the form of a free base), specifically, zastaprazan, a pharmaceutically acceptable salt thereof, a hydrate or solvate thereof, or a mixture thereof may be contained in an amount of about 3.3, 6.6, 13.1, 26.2, or 52.4 mg as zastaprazan (in the form of a free base), and may be contained in an amount of 5 to 40 mg as a zastaprazan citrate salt, specifically 5 mg, 10 mg, 15 mg, 20 mg, or 40 mg as a zastaprazan citrate salt, and more specifically 10 mg or 20 mg as a zastaprazan citrate salt. As described above, the composition of the present invention, even when it contains a low dose (10 mg or 20 mg) of zastaprazan, exhibits a *H. pylori* eradicating effect equivalent to or greater than that of Nexium tablet 40 mg when administered once a day, thereby exhibiting a high healing rate, and has the advantage of ameliorating symptoms such as heartburn, acid reflux, and heartburn/acid reflux within 24 hours of administration and for 7 days.

In the present invention, the composition for bacterial eradication may include one or more pharmaceutical additives consisting of excipients, disintegrants, binders, and lubricants, but is not limited thereto.

In the present invention, the excipient may be one or more of microcrystalline cellulose, lactose monohydrate, anhydrous lactose, sucrose, D-mannitol, starch, corn starch, or light anhydrous silicic acid, but is not limited thereto.

In the present invention, the disintegrant may include starch or modified starch such as sodium starch glycolate, corn starch, potato starch, or pregelatinized starch; clay such as bentonite, montmorillonite, or veegum; cellulose such as hydroxypropyl cellulose or carboxymethyl cellulose; alginates such as sodium alginate or alginic acid; cross-linked celluloses such as croscarmellose sodium; gums such as guar gum or xanthan gum; cross-linked polymers such as crospovidone; effervescent agents such as sodium bicarbonate or citric acid, and the like. These may be used alone or in combination of two or more, but are not limited thereto.

In the present invention, the binder may be one or more selected from the group consisting of hydroxypropyl cellulose, hydroxypropyl methylcellulose, polyvinylpyrrolidone, copovidone, starch, microcrystalline cellulose, colloidal silicon dioxide, mannitol, lactose, polyethylene glycol, and mixtures thereof, but is not limited thereto.

In the present invention, examples of the lubricant may include calcium stearate, glyceryl monostearate, glyceryl palmitostearate, magnesium stearate, sodium lauryl sulfate, sodium stearyl fumarate, zinc stearate, stearic acid, hardened vegetable oils, polyethylene glycol, sodium benzoate, and talc. These may be used alone or in combination of two or more, but are not limited thereto.

In the present invention, the composition for bacterial eradication may be a solid oral formulation, but is not limited thereto.

In the present invention, the solid oral formulation may be one of a tablet, a film-coated tablet, a capsule, powder, a granule, a pill, a troche, an oral jelly, and an oral dissolving film preparation, but is not limited thereto.

In particular, in the case of film-coated tablets, coating agents widely known in the art may be used, but they are not limited thereto.

### Pharmaceutical composition for H. pylori treatment including zastaprazan

The present invention provides a pharmaceutical composition for *Helicobacter pylori* (*H. pylori*) treatment, including zastaprazan, a pharmaceutically acceptable salt thereof, a hydrate or solvate thereof, or a mixture thereof.

The zastaprazan is the same as described above.

The term "treatment" used herein refers to partially or completely alleviating, ameliorating, relieving, inhibiting, or delaying the onset of *H. pylori*, reducing the severity, or reducing the occurrence of one or more symptoms or characteristics by administering the composition according to the present invention.

The above-described contents about the composition for bacterial eradication may be applied to all of the therapeutic compositions as long as they are not contradictory.

### Method of eradicating H. pylori including administering a pharmaceutical composition to a subject in need thereof

The present invention provides a method of eradicating *H. pylori*, the method including administering a pharmaceutically effective amount of a composition including a therapeutically effective amount of zastaprazan, a pharmaceutically acceptable salt thereof, a hydrate or solvate thereof, or a mixture thereof to a subject in need thereof.

The term "pharmaceutically effective amount" used herein refers to an amount effective for eradicating of *H. pylori*, for example, an amount of a composition administered to a subject, which may include all amounts of the composition that prevent the occurrence or recurrence of *H. pylori*, alleviate symptoms, inhibit direct or indirect pathological consequences, prevent metastasis, reduce the rate of progression, alleviate or temporarily reliving a condition, or improve the prognosis. In other words, the pharmaceutically effective amount may be interpreted as encompassing all doses at which *H. pylori* may be eradicated by the composition.

Specifically, in the method of eradicating *H. pylori* according to the present invention, zastaprazan or a pharmaceutically acceptable salt thereof, a hydrate or solvate thereof, or a mixture thereof may be contained in an amount of 1 mg to 100 mg, 2 mg to 60 mg or 3 mg to 40 mg as zastaprazan (in the form of a free base), specifically, zastaprazan or a pharmaceutically acceptable salt thereof, a hydrate or solvate thereof, or a mixture thereof may be contained in an amount of about 3.3, 6.6, 13.1, 26.2, or 52.4 mg as zastaprazan (free base form) or 5 to 40 mg as a zastaprazan citrate salt, specifically 5 mg, 10 mg, 15 mg, 20 mg, or 40 mg as a zastaprazan citrate salt, and more specifically 10 mg or 20 mg as a zastaprazan citrate salt. Preferably, a zastaprazan citrate salt in an amount of 5 to 40 mg, more preferably, a zastaprazan citrate salt in an amount 5 mg, 10 mg, 15 mg, 20 mg, or 40 mg may be administered to a subject once a day to effectively treat the subject by eradicating *H. pylori*.

The term "subject" as used herein refers to a mammal, and specifically, mammals including humans include mammals such as humans, monkeys, cows, horses, dogs, cats, rabbits, rats, and mice, and more specifically, may mean humans. The subject may be a person infected or suspected of being infected with *H. pylori*, due to causes such as a gastroesophageal reflux disease, chronic gastritis, stomach or duodenal ulcers, and stomach cancer.

Here, the term "person infected with *H. pylori*" refers to a person who has been confirmed to have been infected with *H. pylori* through a respiratory or stool test or upper endoscopy due to symptoms such as gastroesophageal reflux symptoms (heartburn, acid reflux, heartburn/acid reflux, etc.), upper abdominal pain, indigestion, or abdominal discomfort (a feeling of fullness of gas, bloating, or burning sensation). The term "person suspected of being infected with *H. pylori*" refers to a case where the above symptoms are experienced.

The above-described contents about the composition for bacterial eradication may be applied to all of the method of bacterial eradication as long as they are not contradictory.

### Use in eradicating H. pylori

The present invention provides a use of a composition including zastaprazan, a pharmaceutically acceptable salt thereof, a hydrate or solvate thereof, or a mixture thereof in eradicating *H. pylori*.

The above-described contents about the composition for bacterial eradication may be applied to all of the use in bacterial eradication as long as they are not contradictory.

### Use in preparing a drug for H. pylori eradication

The present invention provides a use of a composition including zastaprazan, a pharmaceutically acceptable salt thereof, a hydrate or solvate thereof, or a mixture thereof in preparing a drug for *H. pylori* eradication.

The composition of the present invention for preparing a drug may be mixed with an acceptable carrier and the like, and may further contain other agents.

The above-described contents about the composition for bacterial eradication may be applied to all of the use in preparing a drug for *H. pylori* eradication as long as they are not contradictory.

Hereinafter, the present invention will be described in more detail using preparation examples and examples. It will be apparent to those skilled in the art that these preparation examples and examples are only intended to explain the present invention more specifically and that the scope of the present invention is not limited by them.

The active ingredient used in the following preparation examples and examples is a zastaprazan citrate salt, which is named as zastaprazan or the code name JP-1366 for the sake of convenience.

### Preparation Examples

### Preparation Example 1. Preparation of zastaprazan citrate salt

Azetidin-1-yl{8-[(2,6-dimethylbenzyl)amino]-2,3-dimethylimidazo[1,2-a]pyridin-6-yl}methanone citrate salt was obtained according to the process described below. Specifically, azetidin-1-yl{8-[(2,6-dimethylbenzyl)amino]-2,3-dimethylimidazo[1,2-a]pyridin-6-yl}methanone was obtained as described in Korean Patent Publication No. 10-1777971. The NMR analysis results of azetidin-1-yl{8-[(2,6-dimethylbenzyl)amino]-2,3-dimethylimidazo[1,2-a]pyridin-6-yl}methanone obtained above are described below.

¹H NMR (400 MHz, CDCl₃); δ7.63 (d, J=1.2 Hz, 1H), 7.13 (dd, J=8.4, 6.8 Hz, 1H), 7.06-7.04 (m, 2H), 6.42 (d, J=1.2 Hz, 1H), 4.86-4.84 (m, 1H), 4.41-4.28 (m, 4H), 4.37 (d, J =4.4 Hz, 2H), 3.75-3.69 (m, 1H), 2.43-2.34 (m, 13H).

Next, the azetidin-1-yl{8-[(2,6-dimethylbenzyl)amino]-2,3-dimethylimidazo[1,2-a]pyridin-6-yl}methanone obtained above was mixed with an alcohol solvent (isopropyl alcohol, IPA), and the resulting mixture was stirred and dried under vacuum at about 30 °C to 35 °C to obtain a dried product. About 10 g of the dried product was taken and stirred with about 167 g of acetone. To this, a solution prepared by dissolving citric acid (about 5 g) in acetone (about 33 g) was slowly added dropwise over 60 minutes, and the resulting mixture was stirred at the same temperature for one hour. The mixture was cooled to about 20 °C to 25 °C and stirred for one additional hour, and the resulting solid was filtered, washed with acetone, and dried under vacuum to obtain an azetidin-1-yl{8-[(2,6-dimethylbenzyl)amino]-2,3-dimethylimidazo[1,2-a]pyridin-6-yl}methanone citrate salt. The NMR analysis results of the azetidin-1-yl{8-[(2,6-dimethylbenzyl)amino]-2,3-dimethylimidazo[1,2-a]pyridin-6-yl}methanone citrate salt (zastaprazan citrate salt) obtained above are described below.

¹H NMR (400 MHz, MeOD); δ 7.90 (s, 1H), 7.06-7.15 (m, 3H), 6.77 (s, 1H), 4.50 (t, J=7.2Hz, 2H), 4.45 (s, 2H), 4.24 (t, J=7.2 Hz, 2H), 2.80 (d, J=15.6Hz, 2H), 2.70 (d, J=12.0, 2H), 2.39-2.44 (m, 11H), 2.35 (s, 3H).

### Preparation Example 2. Preparation of JP-1366 capsule 5 mg (zastaprazan citrate salt 5 mg)

5 mg of the azetidin-1-yl{8-[(2,6-dimethylbenzyl)amino]-2,3-dimethylimidazo[1,2-a]pyridin-6-yl}methanone citrate salt (zastaprazan citrate salt) obtained according to Preparation Example 1 was mixed with 220.8 mg of D-mannitol, 13.0 mg of croscarmellose sodium, and 1.2 mg of magnesium stearate to obtain a mixture. The mixture was filled into a hard capsule No. 1 used as a capsule base to prepare a capsule preparation.

The capsule preparation prepared in this way is referred to as 'JP-1366 Capsule 5 mg.'

### Preparation Example 3. Preparation of JP-1366 tablet 20 mg (zastaprazan citrate salt 20 mg)

20 mg of the azetidin-1-yl{8-[(2,6-dimethylbenzyl)amino]-2,3-dimethylimidazo[1,2-a]pyridin-6-yl}methanone citrate salt (zastaprazan citrate salt) obtained according to Preparation 1 was mixed with 133.4 mg of microcrystalline cellulose, 6.4 mg of sodium stearyl fumarate, 40.0 mg of anhydrous lactose, 6.0 mg of croscarmellose sodium, and 4.2 mg of magnesium stearate to obtain a mixture. The mixture was compressed directly to obtain tablets. Then, 8.0 mg of Opadry 03B54445 pink was used for coating to prepare a film-coated tablet.

The film-coated tablet prepared in this way is referred to as 'JP-1366 Tablet 20 mg.'

### Preparation Example 4. Preparation of JP-1366 capsule 20 mg (zastaprazan citrate salt 20 mg)

20 mg of the azetidin-1-yl{8-[(2,6-dimethylbenzyl)amino]-2,3-dimethylimidazo[1,2-a]pyridin-6-yl}methanone citrate salt (zastaprazan citrate salt) obtained according to Preparation Example 1 was mixed with 181.4 mg of D-mannitol, 12.4 mg of croscarmellose sodium, and 1.2 mg of magnesium stearate to obtain a mixture. The mixture was filled into a hard capsule No. 1 used as a capsule base to prepare a capsule preparation.

The capsule preparation prepared in this way is referred to as 'JP-1366 Capsule 20 mg.'

### Preparation Example 5. JP-1366 Capsule 20 mg Placebo

A zastaprazan capsule 20 mg placebo was prepared in the same manner as in Preparation Example 4, except that the main ingredient zastaprazan citrate salt was not used. The capsule preparation prepared in this way is referred to as 'JP-1366 capsule 20 mg Placebo'.

### Example 1. Anti-H. pylori in vitro efficacy test

An experiment was performed to determine whether zastaprazan has growth inhibition or urease activity inhibition effects on *H. pylori* under *in vitro* experimental conditions.

### 1. Test substances

### A. Drugs

Table 1 shows the drugs used in the *in vitro* experiment.

**[Table 1]**

| Drugs | CAS No. | Manufacturer | Purity |
|---|---|---|---|
| Zastaprazan (JP-1366) | 2133852-18-1 | Jeil Pharmaceutical | 99.97% |
| Omeprazole | 73590-58-6 | TCI | >98.0%(HPLC)(T) |
| Amoxicillin Trihydrate | 61336-70-7 | TCI | >98.0%(T) |
| Clarithromycin | 81103-11-9 | TCI | >98.0%(T) |
| Fexuprazan | 1902954-60-2 | Chemscene | 99.58% |

### B. Reagents

Table 2 shows the reagents used in the *in vitro* experiment.

**[Table 2]**

| Reagents | CAS No. | Manufacturer | Purity |
|---|---|---|---|
| Brain Heart Infusion | 237500 | BD (Difco) | N/A |
| Muller-Hinton broth | 275730 | BD (Difco) | N/A |
| Bacto-agar | 214010 | BD (Difco) | N/A |
| Laked Horse Blood | N/A | Oxoid | N/A |
| Isovitalex | N/A | BD (BBL) | N/A |
| Horse serum | N/A | Sigma-Aldrich | hemoglobin, ≤20 mg/dL |
| CampyGen | N/A | Oxoid | N/A |

### C. Bacterial strain

*Helicobacter pylori* (ATCC43504): Type strain for antibiotic susceptibility testing according to the Clinical & Laboratory Standards Institute (CLSI) guidelines.

*Helicobacter pylori* 26695 (ATCC700329): A strain isolated from a British patient known to be associated with the development of gastric cancer. The strain is cytotoxin associated gene (CagA)-positive and vacuolating cytotoxin (VacA)-positive and has a well-known genetic background, so it is mainly used for research purposes.

*Helicobacter pylori* SS1 (mouse colonizing strain): A strain known as Sydney strain. The strain is CagA-positive and VacA-positive and has been confirmed to infect and colonize in C57BL/6 mice. The strain is used in animal testing.

### D. Instruments

Table 3 shows the instruments used in the *in vitro* experiment.

**[Table 3]**

| Equipment | Manufacturer | Specifications |
|---|---|---|
| 37°C incubator | Visionbionex | Vision/VS-1203P3V |
| Microcentrifuge | Gyrozen | 1730R |
| Spectrophotometer | BIO-RAD | BR170-2525 |
| Microplate reader | Molecular Devices | SpectraMaxABS |

### 2. Medium preparation

### A. Culture medium

A liquid medium was prepared using brain heart infusion (BHI; Difco Inc., Sparks, MD, USA), 7% differentiation medium (horse serum; Sigma CO., LTD., St. Louis, USA), and 0.4% isovitalex (BBL Inc., Sparks, MD, USA), and a solid medium was prepared by adding 7% laked horse blood (Oxoid Inc., Basingstoke, Hants, UK), 0.4% isovitalex, and 1.5-2% bacto-agar (Difco Inc., Sparks, MD, USA) to BHI.

### B. Medium for measuring minimum inhibitory concentration (MIC)

The medium was prepared using Müller-Hinton broth (MH, Difco Inc., Sparks, MD, USA) [w/7% differentiation medium (Sigma CO., LTD., St. Louis, USA), 0.4% isovitalex (BBL Inc., Sparks, MD, USA)].

### 3. In vitro efficacy test - MIC and minimum bactericidal concentration (MBC) of zastaprazan (JP-1366) against H. pylori

### (1) Method

An antibiotic susceptibility testing was performed using the microdilution method according to the CLSI guideline. Two-fold serial dilution was performed in 12 steps in a 48-well plate with the highest concentration of 128 µg/mL for amocixillin and clarithromycin and 256 µg/mL for omeprazole. The bacteria were inoculated into wells containing only medium without antibiotics to prepare the positive control group, and the wells containing only medium without antibiotics or inoculated bacteria were used as the negative control group. The concentration range of the test substances, zastaprazan (JP-1366) and fexuprazan, was determined through preliminary experiments. *H. pylori* stored in a deep freezer (-70 °C or lower) was thawed, inoculated onto BHI agar (w/7% laked horse blood and 0.4% isovitalex), cultured at 37 °C under microaerobic conditions of 5% O₂, 10% CO₂, and 85% N₂ gas for three days, and then subcultured onto a new medium. The cultured bacteria were harvested, suspended in PBS, inoculated into each well at a concentration of 5 x 10⁵ cfu/mL, and then cultured at 37 °C under microaerobic conditions of 5% O₂, 10% CO₂, and 85% N₂ gas for three days. The lowest concentration at which no bacterial growth was observed was determined as the MIC. Thereafter, 10 µL of the culture solution from each well at different concentrations was spotted onto BHI agar (w/7% laked horse blood and 0.4% isovitalex) containing no antibiotics and cultured for three days at 37 °C under microaerobic conditions. The lowest concentration at which no bacteria growth occurred was determined as the MBC.

### (2) Experimental results and analysis

To determine the MIC, visual inspection was performed and the absorbance was measured at 600 nm, and the growth was confirmed based on the absorbance of the negative control group containing only the medium. To determine the MBC, 10 µl of the culture solution from each well was spotted on a solid medium containing no antibiotics or test substances after the MIC determination, and bacterial growth was examined.

With regard to the suitability of the system in this test, quality control was carried out base on the MIC value of amoxicillin against the type strain, *H. pylori* ATCC43504.

All tests were performed in duplicate, and the results were determined by conducting the experiments in triplicate.

Referring to Table 4 below, since the solubility of zastaprazan (JP-1366) was low, the MIC analysis was difficult (poor solubility, formation of suspension, interference in visual/absorbance analysis), so the inhibitory effect on the *H. pylori* strains was confirmed through the MBC index.

It was confirmed that the test substance, zastaprazan (JP-1366), exhibited an inhibitory effect against three types of *H. pylori* strains at least 2 times and up to 16 times better than that of omeprazole and fexuprazan in terms of MBC.

**[Table 4]**

| Strains | JP1366 | | Amoxicillin | | Clarithromycin | | Omeprazole | | Fexuprazan | |
|---|---|---|---|---|---|---|---|---|---|---|
| | MIC | MBC | MIC | MBC | MIC | MBC | MIC | MBC | MIC | MBC |
| 43504 | nd | 8~32 | 0.016~0.032 | 0.032 | 0.032 | 0.063 | 32 | 64 | 64 | 64~128 |
| 26695 | nd | 16~32 | 0.016~0.032 | 0.032 | 0.063~0.125 | 0.125 | 32~64 | 64~128 | 64 | 64~128 |
| SS1 | nd | 16~323 | 0.125~0.25 | 0.25 | 0.032 | 0.125 | 32~64 | 64~128 | 64 | 64~128 |

### Example 2. Anti-H. pylori efficacy test in patients with erosive gastroesophageal reflux disease (phase 2 clinical trial)

### 1. Subject selection

### H. pylori testing

*H. pylori* testing was performed by 13C-urea breath test (13C-UBT), gastroscopy (*Campylobacter*-like organism test, CLO), or tissue biopsy to confirm *H. pylori* positivity/negativity. The testing was performed at the screening visit (Visit 1) or the randomization visit (Visit 2), and results from the same institution within 29 days prior to the randomization visit (Visit 2) could be substituted.

### Inclusion criteria

The subjects were required to meet all of the following inclusion criteria to participate in this clinical trial, unless otherwise specified.
1) Adult men and women aged 19 to 75 as of the date of written consent
2) Those who experienced heartburn or acid reflux symptoms within seven days prior to the screening visit (Visit 1)

**[Table 5]**

| | |
|---|---|
| Heartburn | • Heartburn or burning sensation inside the breastbone |
| | • Pain inside the breastbone |
| | • Soring or burning sensation at the center of the epigastric region (solar plexus) |
| | • Pain at the center of the epigastric region (solar plexus) |
| Acid reflux | • Reflux of acid (gastric acid) |
| | • Symptoms of reflux of gastric contents (gastric acid or food) through the esophagus |

3) Those diagnosed with grade A or higher erosive gastroesophageal reflux disease according to the Los Angeles Classification at the same institution through upper gastrointestinal endoscopy within 29 days prior to the randomization visit (Visit 2)

**[Table 6]**

| LA grade | Criteria |
|---|---|
| A | One or more mucosal breaks of the esophagus < 5 mm in length |
| B | One or more mucosal breaks of the esophagus ≥ 5 mm in length |
| C | Mucosal breaks of the esophagus that are continuous but involve less than or equal to 75% of the circumference of the esophagus |
| D | Mucosal breaks of the esophagus that involve at least 75% of the esophageal circumference |

4) Those who are able to complete the questionnaire and subject diary
5) Those who voluntarily agreed in writing to participate in this clinical trial

### Exclusion criteria

Those who met any of the following criteria were excluded from this clinical trial.
1) Excluded diseases
   (1) Those with Barrett's esophagus exceeding 3 cm or with significant dysplastic changes as determined by upper gastrointestinal endoscopy at the time of screening visit (Visit 1)
   (2) Eosinophilic esophagitis (Those who are found negative in esophageal biopsy may be included.)
   (3) Those with primary esophageal motility disorder or esophageal stricture
   (4) Gastroesophageal varix
   (5) Those with gastrointestinal bleeding or other abnormal bleeding as determined by upper gastrointestinal endoscopy at the time of screening visit (Visit 1)
   (6) Those who have undergone gastric acid secretion suppression surgery or gastric or esophageal surgery. However, those who correspond to the following may be included.
      ① Appendectomy, cholecystectomy, polypectomy
      ② Endoscopic malignant tumor resection or endoscopic resection of early gastric cancer (endoscopic mucosal resection (EMR) and endoscopic submucosal dissection (ESD)) that has been healed and has not recurred for more than five years from the date of diagnosis
   (7) Those with active duodenal ulcer, gastric ulcer, or pancreatitis
   (8) Zollinger-Ellison syndrome
   (9) Those with irritable bowel syndrome (IBS) or inflammatory bowel disease (IBD)
   (10) Those with warning symptoms (e.g. dysphagia, severe dysphagia, bleeding, weight loss, anemia, bloody stool) that may be suspected of being a malignant disease of the gastrointestinal tract at the investigator's discretion (However, those who are found negative after confirming the presence of a tumor by upper gastrointestinal endoscopy may be included.)
2) Medical history and comorbidity
   (1) Those with clinically significant liver, kidney, nervous system, respiratory system, endocrine system, hematological tumor, cardiovascular system, or urinary system diseases
   (2) Those with a history of malignant tumor within five years prior to the screening visit (Visit 1) (However, those who have been healed and have passed more than five years from the date of diagnosis without recurrence may be included, but those with a history of malignant tumor of the digestive system and tumor resection (e.g., gastric resection, colectomy, etc.) excluding cases falling under (1)-(6) may not be included.)
   (3) Those who have had experience with alcohol or drug abuse within one year prior to the screening visit (Visit 1)
   (4) Those with a confirmed history of human immunodeficiency virus (HIV)
   (5) Those diagnosed with a mental illness that may affect the implementation of this clinical trial (e.g., schizophrenia, dementia)
3) Laboratory test results (at screening visit)
   (1) Those whose serum AST, ALT, ALP, γ-GT, or total bilirubin levels are more than twice the normal upper limits
   (2) Those whose serum BUN or serum creatinine levels are more than twice the normal upper limits
   (3) Active hepatitis B or C (Those who are detected with no virus may be included.)
4) Drug intake/treatment history
   (1) Those who are taking drugs that are prohibited from concomitant use or are expected to take them during the clinical trial period.
5) Hypersensitivity history
   (1) Patients with hypersensitivity to clinical trial drugs, Nexium tablet (Esomeprazole) components, benzimidazoles, penicillin antibiotics, and macrolide antibiotics, and a history thereof
6) Others
   (1) Pregnant women, breastfeeding women, or women who have tested positive in a pregnancy test, or women with childbearing potential or men who plan to become pregnant during this clinical trial period
   (2) Female subjects and female partners of male subjects with childbearing potential who have not undergone infertility surgery and who do not agree to use the contraceptive methods below during the clinical trial period
   (3) Those who have been administered with other clinical trial drugs within one month prior to the screening visit (Visit 1)
   (4) Those who are judged by the investigator to be unsuitable for participation in this clinical trial other than those mentioned above

### 2. Clinical trial method

This clinical trial was a randomized, double-blind, active-controlled, multicenter, phase 2 clinical trial targeting patients with erosive gastroesophageal reflux disease. After the subjects gave written consent to participate in the clinical trial, a screening test was conducted. When the subjects were taking drugs that could affect the gastric mucosa at the time of screening, an upper gastrointestinal endoscopy was performed after a 2-week drug-free period (a 4-week drug-free period for potassium-competitive acid blockers (P-CABs) or proton pump inhibitors (PPIs)). Subjects who met the inclusion/exclusion criteria based on the screening test results were randomly assigned to test group 1, test group 2, or the control group at a ratio of 1:1:1. At this time, the subjects were stratified by grade (A, B, C, D) according to the LA classification system classified by upper gastrointestinal endoscopy. Randomly assigned subjects took the clinical trial drug once a day according to the assigned administration group for four weeks. After four weeks of administration of the clinical trial drug, the subjects visited the clinical trial institution to undergo an upper gastrointestinal endoscopy to assess whether they were healed. Healed subjects underwent a Safety Follow-Up (F/U) two weeks later, and unhealed subjects were administered the clinical trial drug for additional four weeks (a total of 8 weeks of administration) and visited the clinical trial institution to undergo an upper gastrointestinal endoscopy. Regardless of whether they were healed, a Safety F/U was performed 2 weeks later.

### 3. Dosage, administration period, and administration method

### A. Dosage

- Test drug 1: JP-1366 5 mg 2 capsules (Zastaprazan citrate salt 10 mg)
- Test drug 2: JP-1366 20 mg 1 capsule (Zastaprazan citrate salt 20 mg)
- Control drug: Esomeprazole magnesium trihydrate 44.5 mg (Esomeprazole 40 mg, Nexium tablet 40 mg 1 tablet)

### B. Administration period

Trial period per subject: Up to 14 weeks
- Screening period: 15 days or 29 days
- Administration period: 4 weeks (or 8 weeks)
- Safety F/U period: 2 weeks after the last administration of the clinical trial drug

### C. Administration method

Depending on the randomly assigned administration group, the subjects were orally administered with the clinical trial drug once a day at regular intervals on an empty stomach for four weeks (or 8 weeks*) from the day the clinical trial drug was provided.

*Only for subjects whose mucosal breaks have not been healed after four weeks of clinical trial drug administration, an additional 4-week administration period was given.

### 4. Efficacy endpoints

### A. Primary efficacy endpoint:

Cumulative healing rate of mucosal breaks up to week 8 after the administration of the clinical trial drug (%)

*Recovery of erosion to normal mucosa as found in upper gastrointestinal endoscopy

### B. Secondary efficacy endpoints:

① Mucosal break healing rate (%) up to week 4 after administration of clinical trial drug
② Symptom evaluation through subject diary
   A. Evaluation of symptoms (heartburn, acid reflux, heartburn/acid reflux) within 24 hours after the administration of the clinical trial drug
   B. Evaluation of symptoms (heartburn, acid reflux, heartburn/acid reflux) for seven days after the administration of the clinical trial drug
   C. Days of achieving complete remission (CR, disappearance of heartburn and acid reflux for seven days) after the administration of the clinical trial drug
   D. Proportion of symptom-free days (heartburn, acid reflux, heartburn/acid reflux) for 4 and 8 weeks after the administration of the clinical trial drug (daytime, nighttime, daytime/nighttime)
③ Changes in frequency and severity of major symptoms at week 4 and week 8 of the clinical trial drug administration compared to the gastroesophageal reflux disease questionnaire (RDQ) baseline
④ Changes in total score at week 4 and week 8 of the clinical trial drug administration compared to the GERD Health-Related Quality of Life (GERD-HRQL) baseline

### 5. Safety endpoints

(1) Adverse reactions
(2) Vital signs
(3) Laboratory tests
(4) Electrocardiogram (12-laed ECG)
(5) Physical examination

### 6. Statistical analysis method

### A. Primary efficacy endpoint

Cumulative healing rate of mucosal breaks up to week 8 after the administration of the clinical trial drug (%)

The proportion of subjects whose erosions recovered to normal mucosa from the first administration of the clinical trial drug up to week 8 and the two-sided 95% confidence intervals were presented by administration group. To compare each test group with the control group (JP-1366 10 mg vs Nexium tablet 40 mg, JP-1366 20 mg vs Nexium tablet 40 mg), the Cochran-Mantel-Haenszel method corrected for baseline LA grade (A, B, C, D) as a stratification factor was used to obtain the lower limit of each two-sided 95% confidence interval.

### B. Secondary efficacy endpoints

① Mucosal break healing rate (%) up to week 4 after administration of clinical trial drug
   The proportion of subjects whose erosions recovered to normal mucosa from the first administration of the clinical trial drug up to week 4 and the two-sided 95% confidence intervals were presented by administration group. To compare each test group with the control group (JP-1366 10 mg vs Nexium tablet 40 mg, JP-1366 20 mg vs Nexium tablet 40 mg), the Cochran-Mantel-Haenszel method corrected for baseline LA grade (A, B, C, D) as a stratification factor was used to obtain the lower limit of each two-sided 95% confidence interval.
② Symptom evaluation through subject diary
   A. Evaluation of symptoms (heartburn, acid reflux, heartburn/acid reflux) within 24 hours after the administration of the clinical trial drug: Comparison of each test group with the control group for the mean evaluation of symptoms (heartburn, acid reflux, heartburn/acid reflux) within 24 hours after the first administration of the clinical trial drug was analyzed using an analysis of covariance (ANCOVA) model with baseline LA grade (A, B, C, D) as a covariate.
   B. Evaluation of symptoms (heartburn, acid reflux, heartburn/acid reflux) for seven days after the administration of the clinical trial drug: Comparison of each test group with the control group for the mean evaluation of symptoms (heartburn, acid reflux, heartburn/acid reflux) for seven days after the first administration of the clinical trial drug was analyzed using an ANCOVA model with baseline LA grade (A, B, C, D) as a covariate.
   C. Days of achieving CR (disappearance of heartburn and acid reflux for seven days) after the administration of the clinical trial drug: When the symptom score is 0 for seven consecutive days after the first administration of the clinical trial drug, it was defined as CR (disappearance of heartburn and acid reflux for seven days), and the event was based on the first CR. When there was no CR, the case was considered as censoring. Kaplan-Meier plots and medians and their two-sided 95% confidence intervals were presented by administration group, and the comparison of each test group to the control group was analyzed using the stratified log-rank test corrected for the baseline LA grade as a stratification factor.
   D. Proportion of symptom-free days (heartburn, acid reflux, heartburn/acid reflux) for 4 and 8 weeks after the administration of the clinical trial drug (daytime, nighttime, daytime/nighttime): Comparison of each test group with the control group in the proportion of symptom-free days (heartburn, acid reflux, heartburn and acid reflux) for 4 and 8 weeks after the first administration of the clinical trial drug in daytime, nighttime, daytime and nighttime, respectively, is analyzed using an ANCOVA model with baseline LA grade (A, B, C, D) as a covariate.
③ Comparison of each test group with the control group in the changes in frequency and severity of major symptoms at week 4 and week 8 of the clinical trial drug administration compared to the gastroesophageal RDQ baseline was analyzed using an ANCOVA model with baseline LA grade (A, B, C, D) as a covariate. The same statistical analysis as above was performed on the changes in the last results, excluding the baseline compared to the baseline.
④ Comparison of each test group with the control group in the changes in total score at week 4 and week 8 of the clinical trial drug administration compared to the quality of life evaluation (GERD-HRQL) baseline was analyzed using an ANCOVA model with baseline LA grade (A, B, C, D) as a covariate. The same statistical analysis as above was performed on the changes in the last results, excluding the baseline compared to the baseline.

### C. Safety endpoints

For subjects who experienced adverse reactions (or adverse drug reactions) at least once, the number of occurrences and occurrence rates and their two-sided 95% confidence intervals were presented by administration group, and the Chi-square test or Fisher's exact test was performed to test whether there is a difference in the occurrence rates of adverse reactions (or adverse drug reactions) between the control group and each test group. In addition, the latest version of Medical Dictionary for Regulatory Activities (MedDRA) was used to present the data coded as System Organ Class (SOC) and Preferred Term (PT). Serious adverse reactions, adverse reactions that caused discontinuation of clinical trial drug administration, and adverse reactions that caused death were also summarized as SOC and PT using the latest version of MedDRA. For laboratory tests, vital signs, and electrocardiograms (12-lead ECG), descriptive statistics (number of subjects, mean, standard deviation, median, minimum, maximum), the changes at each visit and from the baseline were presented as continuous data, and contingency tables were created for categorical data. For laboratory tests and electrocardiograms (12-lead ECG), the changes from baseline after the administration of the clinical trial drug were summarized by each evaluation time point in the form of a shift table, categorized into normal or clinically insignificant abnormal (Normal or Abnormal NCS) and clinically significant abnormal (Abnormal CS). For physical examinations, a list of subjects who were normal or clinically insignificant abnormal (Abnormal NCS) at baseline but changed to clinically significant abnormal (Abnormal CS) after the administration of the clinical trial drug was presented.

### 7. Results of phase 2 clinical trial

### A. Confirmation of amelioration of symptoms

In the evaluation of all symptoms (heartburn, acid reflux, heartburn/acid reflux) within 24 hours and for seven days after the administration of the clinical trial drug, it was confirmed that all symptoms tended to be ameliorated within 24 hours and for seven days after the administration of the test drugs 1 and 2 which are clinical trial drug.

### B. Confirmation of healing rate

### (1) H. pylori-positive group (infected group)

The JP-1366 20 mg (Zastaprazan 20 mg) administration group exhibited a 100% healing effect at both week 4 and 8, but Nexium 40 mg showed an 87.5% effect (see Table 7).

**[Table 7]**

| | JP-1366 10 mg (N=7) | JP-1366 20 mg (N=5) | Esomeprazole 40 mg (N=8) |
|---|---|---|---|
| Healing rate at week 4 | | | |
| Healing rate, n (%) | 6 (85.71) | 5 (100.00) | 7 (87.50) |

| Healing rate at week 8 | | | |
|---|---|---|---|
| Healing rate, n (%) | 7 (100.00) | 5 (100.00) | 7 (87.50) |

### (2) H. pylori-negative group

It was confirmed that the JP-1366 20 mg (Zastaprazan 20 mg) administration group exhibited the same effect as Nexium 40 mg (see Table 8). Through this, it was clinically confirmed that zastaprazan can show the same effect as the control group, Nexium 40 mg, even at a significantly low dose.

**[Table 8]**

| | JP-1366 10 mg (N=41) | JP-1366 20 mg (N=42) | Esomeprazole40 mg (N=41) |
|---|---|---|---|
| Healing rate at week 4 | | | |
| Healing rate, n (%) | 39 (95.12) | 38 (90.48) | 37 (90.24) |

| Healing rate at week 8 | | | |
|---|---|---|---|
| Healing rate, n (%) | 40 (97.56) | 41 (97.62) | 40 (97.56) |

### (3) Overall healing rate

Considering the results of the *H. pylori*-positive group (infected group) and the *H. pylori*-negative group, in terms of the overall healing rate, the proportion of subjects whose mucosal breaks were completely healed at week 4 after the administration of the clinical trial drug was 93.75% (45/48 people) in the JP-1366 10 mg group, 91.49% (43/47 people) in the JP-1366 20 mg group, and 89.80% (44/49 people) in the esomeprazole 40 mg group.

### (4) Conclusions

Nexium 40 mg showed a higher erosion healing rate in *H. pylori*-negative patients than in *H. pylori*-positive patients, but the pharmaceutical composition according to the present invention exhibited a therapeutic effect on gastroesophageal reflux disease and/or peptic ulcer regardless of *H. pylori* infection, and rather, it showed a tendency to exhibit a higher effect in patients infected with *H. pylori*. This is due to the bacterial eradicating effect of zastaprazan itself contained in the pharmaceutical composition according to the present invention.

### Example 3. Anti-H. pylori efficacy test for patients with erosive gastroesophageal reflux disease (phase 3 clinical trial)

### 1. Subject selection

### H. pylori testing

*H. pylori* testing was performed by 13C-UBT, gastroscopy (*Campylobacter*-like organism test, CLO), or tissue biopsy to confirm *H. pylori* positivity/negativity. The testing was performed at the screening visit (Visit 1) or the randomization visit (Visit 2), and results from the same institution within 29 days prior to the randomization visit (Visit 2) could be substituted.

### Inclusion criteria

The subjects were required to meet all of the following inclusion criteria to participate in this clinical trial, unless otherwise specified.
1. Adult men and women aged 19 or more as of the date of written consent
2. Those who experienced heartburn or acid reflux symptoms within seven days prior to the screening visit and whose severity and frequency of the symptoms correspond to 1) or 2) below:
1) Those who experienced mild or more severe heartburn or acid reflux twice or more a week
2) Those who experienced moderate or more severe heartburn or acid reflux once or more a week

3. Those diagnosed with grade A or higher erosive gastroesophageal reflux disease according to the Los Angeles Classification^{†} through endoscopy within 15 days prior to the randomization
^{†}Los Angeles Classification

**[Table 9]**

| Grade | Endoscopic finding |
|---|---|
| A | One (or more) mucosal break no longer than 5 mm, that does not extend between the tops of two mucosal folds |
| B | One (or more) mucosal break more than 5 mm, that does not extend between the tops of two mucosal folds |
| C | One (or more) mucosal break that is continuous between the tops of two or more mucosal folds, but which involves less than 75% of the circumference |
| D | One (or more) mucosal break which involves at least 75% of the esophageal circumference |

4. Those who fully understand this clinical trial and voluntarily agreed in writing to participate in this clinical trial

### Exclusion criteria

Those who met any one of the following criteria were excluded from this clinical trial.

The subjects must not meet any of the following criteria.
1. Those who are unable to undergo endoscopy
2. Medical history
   1) Those with warning symptoms (e.g. dysphagia, severe dysphagia, bleeding, weight loss, anemia, bloody stool) that may be suspected of being a malignant disease of the gastrointestinal tract (However, those who are found negative after confirming the presence of a malignant disease by endoscopy may be excluded.)
   2) Patients with Eosinophilic esophagitis (Those who are found negative in esophageal biopsy may be excluded.)
   3) Those with gastroesophageal stenosis, gastroesophageal varices, Barrett's esophagus, active peptic ulcer, gastrointestinal bleeding, or gastrointestinal malignancy confirmed by endoscopy
   4) Patients with Zollinger-Ellison syndrome
   5) Those who has a history or is suspected of a history of primary esophageal motility disorder, IBS, or IBD, including pancreatitis, within the past 3 months
   6) Those who have undergone gastric acid secretion suppression surgery or gastric or esophageal surgery (However, those who have a history of appendectomy, cholecystectomy, and polypectomy may be excluded.)
   7) Those with clinically significant diseases such as liver, kidney, cardiovascular, respiratory, endocrine, urinary, neuropsychiatric, and hematological diseases
   8) Those with a history of malignant tumor within five years prior to the time of screening (However, those with a history of malignant tumor in the digestive system are excluded regardless of the period.)
3. Those who show the following abnormalities in laboratory tests conducted at the time of screening for laboratory tests:
   1) ALT or AST > 2.0 x ULN
   2) Total bilirubin > 2.0 x ULN
   3) ALP or GGT > 2.0 x ULN
   4) eGFR R<70 mL/min/1.73 m² (Chronic Kidney Disease Epidemiology Collaboration (CKD-EPI) equation)
   5) Positive results in serum tests (HBsAg, HCV Ab, HIV Ab, Syphilis reagin test)
   6) Those who show clinically significant abnormal findings in electrocardiography
4. Allergy and drug hypersensitivity
   1) Those known with hypersensitivity to components or additives of the clinical trial drug
   2) Those with a history of clinically significant allergic disease (excluding mild allergic rhinitis that does not require administration) or hypersensitivity to other drugs (aspirin, antibiotics, etc.)
5. Contraindicated drugs and treatments
   1) Those who took acid secretion inhibitors such as P-CAB or PPI within two weeks prior to endoscopy at the time of screening
   2) Those who took reflux esophagitis-related drugs (antacids, prokinetic agents, histamine-2 receptor antagonists) twice or more within one week prior to the endoscopy at the time of screening
   3) Those who are unable to stop taking drugs that may cause ulcers, such as aspirin or non-steroidal antiinflammatory drugs (NSAIDs), during the clinical trial period
   4) Those who are taking or need to take drugs that are contraindicated for concomitant use in this clinical trial. However, those who have taken contraindicated drugs may participate after a two-week washout period. When the period corresponding to 5 times the half-life of the contraindicated drug exceeds two weeks, the washout period shall be 5 times the half-life of the drug.
6. Pregnant women and breastfeeding women
7. Subjects and spouses (or partners) who do not use medically acceptable methods of contraception during the entire period of the clinical trial
   1) Use of an intrauterine device with a proven pregnancy failure rate
   2) Use of a double barrier method of contraception (male condom and closure cap, contraceptive diaphragm or cervical cap) and simultaneous use of spermicide
   3) Infertility procedures (vasectomy, salpingectomy and ligation, hysterectomy)
8. Those with a history of clinically significant mental illness, drug or alcohol abuse
9. Those who are judged by the investigator to be unsuitable for participation in this clinical trial other than those mentioned above

### 2. Clinical trial method

Those who voluntarily agreed to participate in this clinical trial underwent a screening test through a screening visit, and only eligible subjects were allowed to participate in the clinical trial based on the inclusion/exclusion criteria. Eligible subjects were randomly assigned to the test group or control group and received the clinical trial drug according to the assigned group at the baseline visit (V2). Visits were made on day 1 and in week 4 during the total four-week treatment period, and the clinical trial drug was administered orally once a day.

### 3. Dosage, administration period, and administration method

### A. Dosage

- Test drug: JP-1366 20 mg 1 capsule (Zastaprazan citrate salt 20 mg)
- Control drug: Esomeprazole magnesium trihydrate 44.5 mg (Esomeprazole 40 mg, Nexium tablet 40 mg 1 tablet)

### B. Administration period

About 12 weeks (±14 days)
1. Screening period: Up to two weeks from the first administration of the clinical trial drug
2. Treatment period: Four weeks (±7 days) after the first administration of the clinical trial drug
3. Follow-up visit: Two weeks (±14 days) after the last administration of the clinical trial drug

### C. Administration method

- The clinical trial drug was administered orally once a day for a total of four weeks at a set time of the day whenever possible, regardless of meals, according to the randomly assigned administration group.

**[Table 10]**

| Administration group | Drugs for clinical trial | | |
|---|---|---|---|
| Test group | JP-1366 20 mg 1 capsule, Esomeprazole placebo 1 tablet | | •◇ |
| Control group | JP-1366 placebo 1 capsule, Esomeprazole 40 mg 1 tablet | | ○◆ |
| •: JP-1366 20 mg | | ○: JP-1366 placebo | |
| ◆: Esomeprazole 40 mg | | ◇: Esomeprazole placebo | |

### 4. Concomitant drugs

### A. Contraindicated concomitant drugs

The following drugs are contraindicated for administration during this clinical trial period.
1. All gastric acid secretion inhibitors other than the clinical trial drug
2. Gastric mucosal protective agents
3. Antacids
4. Prokinetic agents
5. Other gastrointestinal drugs
6. Steroid preparations (However, topical administration, epidural administration, and inhalation administration are permitted.)
7. NSAIDs (However, short-term use of NSAIDs for the purpose of treating acute pain is permitted at the investigator's discretion.)
8. Cholinergics, antipsychotics, antithrombotics (anticoagulants or antiplatelet agents (including aspirin))
9. Drugs known to interact with the clinical trial drug

### B. Concomitant drugs

1. Drugs administered for the treatment of comorbid diseases could be used without changes in the administration method and dosage. However, when the drugs administered for the treatment of comorbid diseases were judged not to affect this clinical trial, changes in administration and dosage were possible. Other drugs could administered concurrently when they were judged not to affect this clinical trial.
2. The use of drugs for pretreatment of endoscopic examinations and UBT was permitted.
5. Results of phase 3 clinical trial

### A. Confirmation of mucosal breaks healing rate

### (1) Confirmation of cumulative healing rate of mucosal breaks in subjects tested H. pylori-positive

The cumulative healing rate of mucosal breaks according to the presence or absence of *H. pylori* infection refers to the proportion of subjects whose mucosal breaks were healed by 4 weeks after the administration of the clinical trial drug on endoscopic examination.

The frequency and percentage of healing rates of mucosal breaks at week 4 after the administration of the clinical trial drug were presented by administration group according to the presence or absence of *H. pylori* infection. The differences between the administration groups (test group-control group) were analyzed using the Cochran-Mantel-Haenszel method corrected with the baseline LA classification method as a stratification factor, and the 95% confidence intervals for the differences were presented.

### Per protocol set (PPS)

As a result of the PPS analysis, which is the main analysis group of this clinical trial, the number of subjects who were tested *H. pylori*-positive was 26 in the test group and 22 in the control group. The proportion of subjects whose mucosal breaks were healed on endoscopic examination at week 4 after the administration of the clinical trial drug was 100.00% (26/26 subjects) in the test group and 81.82% (18/22 subjects) in the control group, showing a statistically significant difference between the two administration groups (p=0.0214).

### Full analysis set (FAS)

According to the FAS analysis results, the number of subjects who were tested *H. pylori*-positive was 28 in the test group and 25 in the control group. The percentage of subjects whose mucosal breaks were healed on endoscopic examination at week 4 after the administration of the clinical trial drug was 96.43% (27/28 subjects) in the test group and 80.00% (20/25 subjects) in the control group. The test group showed an excellent effect with a significant difference compared to the control group at week 4.

**[Table 11]**

| | JP-1366 20 mg | Esomeprazole 40 mg |
|---|---|---|
| Number of subjects | 26 | 22 |
| Number of healed subjects, n (%) | 26 (100.00) | 18 (81.82) |

**[Table 12]**

| | JP-1366 20 mg | Esomeprazole 40 mg |
|---|---|---|
| Number of subjects | 28 | 25 |
| Number of healed subjects, n (%) | 27 (96.43) | 20 (80.00) |

While the specific parts of the present invention have been described in detail above, it is obvious to those skilled in the art that these specific descriptions are merely preferred embodiments and that the scope of the present invention is not limited thereto. Accordingly, the actual scope of the present invention will be defined by the appended claims and their equivalents.

## Claims

1. A composition for *Helicobacter pylori* eradication, comprising zastaprazan, a pharmaceutically acceptable salt thereof, a hydrate or solvate thereof, or a mixture thereof.

2. The composition for *Helicobacter pylori* eradication of claim 1, wherein the pharmaceutically acceptable salt of zastaprazan is a zastaprazan citrate salt.

3. The composition for *Helicobacter pylori* eradication of claim 1, wherein the composition for eradication includes the pharmaceutically acceptable salt of zastaprazan in an amount of 10 mg, 20 mg, or 40 mg.

4. The composition for *Helicobacter pylori* eradication of claim 1, wherein the composition for eradication is administered once to three times a day.

5. The composition for *Helicobacter pylori* eradication of claim 1, wherein the composition for eradication is administered once a day.

6. The composition for *Helicobacter pylori* eradication of claim 1, wherein the composition for eradication is formulated into a solid oral formulation.

7. The composition for *Helicobacter pylori* eradication of claim 6, wherein the solid oral formulation is any one of a tablet, a film-coated tablet, a capsule, powder, a granule, a pill, a troche, an oral jelly, and an oral dissolving film preparation.

8. A pharmaceutical composition for *Helicobacter pylori* (*H. pylori*) treatment, including zastaprazan, a pharmaceutically acceptable salt thereof, a hydrate or solvate thereof, or a mixture thereof.

9. A method of eradicating *H. pylori*, the method including administering a pharmaceutically effective amount of a composition including a therapeutically effective amount of zastaprazan, a pharmaceutically acceptable salt thereof, a hydrate or solvate thereof, or a mixture thereof to a subject in need thereof.

10. A use of a composition including zastaprazan, a pharmaceutically acceptable salt thereof, a hydrate or solvate thereof, or a mixture thereof in eradicating *H. pylori*.

11. A use of a composition including zastaprazan, a pharmaceutically acceptable salt thereof, a hydrate or solvate thereof, or a mixture thereof in preparing a drug for *H. pylori* eradication.
